# EUROPEAN PATENT SPECIFICATION

(11) **EP 3 560 487 B1**
(45) Date of publication and mention of the grant of the patent: **11.11.2026**
(21) Application number: 17888757.6
(22) Date of filing: 26.12.2017
(51) Int. Cl.: A61K 9/20, A61K 9/14, A61K 9/16, A61K 9/48, A61K 31/485, A61K 47/10, A61K 47/26, A61P 1/08, A61P 25/04

(54) **PRODUCTION METHOD FOR FORMULATION HAVING IMPROVED CONTENT UNIFORMITY**
HERSTELLUNGSVERFAHREN FÜR FORMULIERUNG MIT VERBESSERTER GLEICHFÖRMIGKEIT DES GEHALTS
PROCÉDÉ DE FABRICATION D'UNE FORMULATION À UNIFORMITÉ DE TENEUR AMÉLIORÉE

(30) Priority: 26.12.2016 JP 2016250513
(43) Date of publication of application: 30.10.2019
(73) Proprietor: Shionogi & Co., Ltd., Osaka-shi, Osaka 541-0045 (JP)
(72) Inventor: MASHIMO, Akira, Amagasaki-shi Hyogo 660-0813 (JP); ICHIO, Shunji, Amagasaki-shi Hyogo 660-0813 (JP)
(74) Representative: Vossius & Partner Patentanwälte Rechtsanwälte mbB
(86) International application number: PCT/JP2017/046614
(87) International publication number: WO 2018/124062

(56) References cited:
- WO-A1-2012/063933
- WO-A1-2012/063933
- WO-A1-2013/080271
- WO-A1-2013/157754
- WO-A1-2013/164617
- WO-A1-2013/172297
- WO-A1-2015/016256
- JP-A- 2008 094 751
- JP-A- 2008 530 070
- JP-A- 2014 526 496
- JP-A- 2015 519 904

## Description

### Technical Field

The present invention is a manufacturing method of solid formulations comprising a process of mixing the active ingredient and an excipient with a median particle size of 15 times or more, preferably 20 times or more, relative to the median particle size of the said active ingredient. By taking the said manufacturing method, the standard deviation of content on the active ingredient in the formulation can be 5% or less.

### Background Art

Content uniformity is a problem in the case of formulations containing a low content of active ingredient, especially in the case of tablets. That is, it is difficult to prepare tablets with good content uniformity, even if the tablets are produced by the direct compression method, in which the active ingredient and additive with low content are simply mixed and tableted. When a tablet containing a low content of active ingredient need to be produced by direct compression method, the additive need to be mixed in several steps in small amounts with the active ingredient, and the manufacturing process becomes complicated. Therefore, in the case of tablets with such low-content active ingredients, the active ingredient and additive are often granulated to produce the tablet, once the granule is produced, and the granule is pressed to produce the tablet.

When granules are produced, active ingredients and additives are often mixed, granulated with solvents such as water, and then dried for production. However, some active ingredients can be degraded by solvent such as water. In addition, there is a possibility that the crystal form of the active ingredient is changed by solvents such as water or the like, and the active ingredient may be decomposed by the drying process of the granule.

As described above, when the active ingredient is degraded by a solvent such as water, it is not preferable to produce the tablet by the granule compression method, and the direct compression method without using a solvent such as water is preferable. Therefore, a direct compression method, which can produce tablets with good content uniformity and, which does not complicate the manufacturing process is desired.

Patent Literatures 1 and 2 are disclosed as the literature of tablets produced by the direct compression method with a low content of active ingredient. However, in these documents, the particle size of the active ingredient alone is reduced, or the particle size of the additive alone is reduced to make the content uniformity good, but it is not always possible to achieve the objective content uniformity.

Active ingredients that exert medicinal effects at low content include 6,7-unsaturated-7-carbamoylmorphinane derivatives, which are side effect reducing agent of agonists of opioid receptors. Patent Literatures 3 and 4 disclose the said compound and the manufacturing method of producing the said compound. Also, in Patent Literature 5, a formulation containing the said compound is disclosed. However, no content uniformity is disclosed in Patent Literatures 3 to 5.
WO 2013/164617 A1 discloses pharmaceutical compositions for the treatment of pain, which compositions comprise a mixture comprising: (a) microparticles of sufentanil, or a pharmaceutically acceptable salt thereof, which microparticles are presented on the surfaces of larger carrier particles; (b) a water-soluble weak base; and (c) a compound which is a weak acid.

### Citation list

### Patent literature

[Patent Literature 1] International Publication No. 2008/072354
[Patent Literature 2] Japanese Patent Laid-Open No.2015-530355
[Patent Literature 3] International Publication No. 2006/126637
[Patent Literature 4] International Publication No. 2012/063933
[Patent Literature 5] International Publication No. 2013/172297

### Summary of invention

### Problems to be solved by the Invention

The problem of the present invention is to provide a manufacturing method of a formulation which is simple and has high content uniformity of the active ingredient by the direct compression method, and the formulation.

### Means for Solving the Problems

In order to solve the above-mentioned problems, the inventors conducted intense research, focusing on the difference in the median particle size between the active ingredient and the excipient, and found that a solid formulation with a standard deviation of the active ingredient content in the formulation within 5% can be produced by mixing excipients with a median particle size of 15 times or more for the median particle size of the active ingredient, even if the amount of the active ingredient is small, leading to the completion of the present invention (Hereinafter, a formulation thus accomplished by the present invention is sometimes referred to as the "present formulation".). The invention is set out in appended claims 1 to 26.

Specifically, the present invention relates to the following:
(1) A process for manufacturing a solid formulation as defined in process claims 1 to 23.
(2) A solid formulation as defined in any of claims 24 to 26 produced by the process for manufacturing, according to any one of appended claims 1 to 23.

### Effect of the Invention

According to the present invention, the content uniformity of the active ingredient is high. A result of the determination on the content of the solid formulations of the present invention shows that the standard deviation of the content of the active ingredient is 5% or less, preferably 3% or less.

### Mode for carrying out the invention

In the present formulation, the active ingredient may be used in a medicine. Especially, active ingredients with a small content of active ingredients in the formulation or those that are unstable to water can exert the effects of the present invention. Here, the term "water-labile active ingredient" refers to an active ingredient that breaks down by water.

As active ingredient used in the present formulation,
the compound represented by the formula (IA): 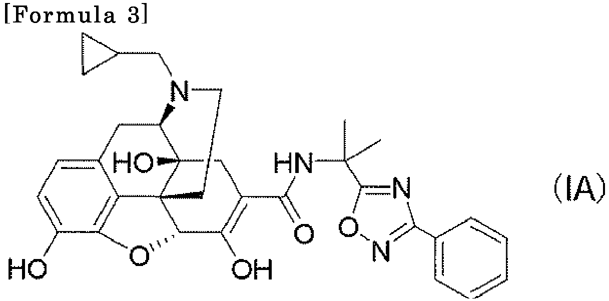
, or its pharmaceutically acceptable salts, or their solvate are used.

The active ingredient can be used alone or in combination with other pharmaceuticals. In addition, these drugs are administered in a known appropriate amount as determined according to the disease, age, and the like of the patient.

It should be noted that the active ingredient not only include compounds but also pharmaceutically acceptable salt of the compounds or their solvate.

The content of the active ingredient in the present formulation may be such that it produces a drug effect. Specifically, it is not more than 10% by weight, preferably between 0.001 and 10% by weight, more preferably not more than 5% by weight, more preferably between 0.005 and 5% by weight, particularly preferably not more than 1% by weight, very preferably between 0.01 and 1% by weight, with respect to the total amount of the formulation.

As an active ingredient in the present formulation the compound represented by the formula (IA): 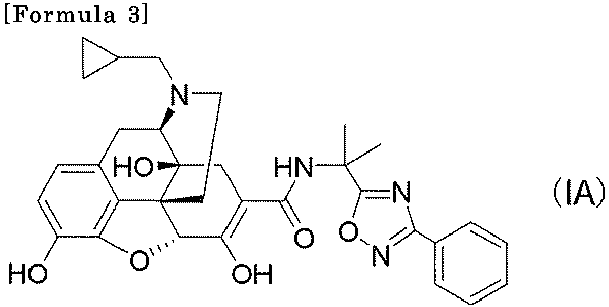 , or its pharmaceutically acceptable salts, or their solvate are used. It is preferably p-toluenesulfonic acid salt, acetate or hydrochloride of the compound represented by the formula (IA) or its solvate as an active ingredient, more preferably p-toluenesulfonic acid salt of the compound represented by the formula (IA) or its solvate as an active ingredient.

The formulation of compounds represented by the formula (IA), its pharmaceutically acceptable salts or their solvate, and p-toluenesulfonic acid salts of compounds represented by the formula (IA) or its solvate are disclosed in the International Publication No. 2006/126637 and International Publication No. 2012/063933.

The content of the compound represented by the formula (IA) in the present formulation, in particular, the compound p-toluenesulfonic acid salt represented by the formula (IA) or the solvate of the salt, may be in such amount as to produce a medicinal effect. Specifically, it is not more than 10% by weight, preferably between 0.001 and 10% by weight, more preferably not more than 5% by weight, more preferably between 0.005 and 5% by weight, particularly preferably 1% or less by weight, very preferably between 0.01 and 1% by weight, with respect to the total amount of the formulation.

In the present formulation, the upper limit of the median particle size of the active ingredient, which is also related to the median particle size with the active ingredient, is 10 µm or less, preferably 8 µm or less, and more preferably 5 µm or less. On the other hand, in the formulation of the present invention, the lower limit of the median particle size of the active ingredient is preferably 0.05 µm or more, and more preferably 0.1 µm or more, although there is no particular limitation.

The dose of p-toluenesulfonic acid salt of the compound represented by the formula (IA) in the formulation of the present invention should be set in consideration of the age, body weight, type and degree of disease, administration route, and the like of the patient.

The formulations contain excipients. Excipients have the highest percentage of inclusion among the additives of the formulations of the present invention. As excipients, excipients listed in the Japanese Pharmacopoeia, the Japanese Pharmaceutical Codex or the Japanese Pharmaceutical Excipients and the like can be used. Specifically, sugar alcohols such as D-mannitol, xylitol, sorbitol, maltitol, lactitol, oligosaccharide alcohols, xylose, glucose, fructose, maltose, lactose, sucrose (sucrose), isomerized sugar, syrup, purified sucrose, sucrose, granular purified sucrose, anhydrous lactose, granular sucrose • starch, and the like, hemidigestive starch, sugar hydrate, powder sugar, crystalline cellulose, pullulan, β-cyclodextrin, amino ethyl sulfonic acid, American powder; sodium chloride; sodium citrate; glycine; calcium gluconate; L-glutamine; tartrate; potassium hydrogen tartrate; ammonium carbonate; dextrin; calcium lactate; povidone; macrogol (polyethylene glycol) 1500; macrogol 4000; macrogol 6000; citric anhydride, DL-malate, sodium hydrogen phosphate, sodium dihydrogen phosphate, L-aspartate, sodium calmelose, sodium hydrous silicon, glycerophosphate, calcium glycerophosphate, calcium silicate, magnesium silicate, synthetic aluminium anhydride, wheat flour, wheat germ oil, rice flour, rice starch, cellulose acetate, titanium oxide, dihydroxy aluminium aminoacetate, calcium tertiary phosphate, talc, calcium carbonate magnesium carbonate; natural aluminum silicate; corn starch; corn starch granules; potato starch; hydroxypropyl cellulose; calcium hydrogenphosphate anhydride; calcium hydrophosphate granules, calcium dihydrogen phosphate and the like are included, preferably lactose and crystalline cellulose.

The content of excipients in the present formulation is 50-99.99 wt%, preferably 60-99.99 wt%, and more preferably 70-99.99 wt%, relative to the total amount of the formulations.

In the present formulation, the difference between the median particle size of the active ingredient and the median particle size of the excipient results in high content uniformity. That is, we found that the larger these differences, the smaller the standard deviation of the active ingredient content. That is, if the median particle size of the excipient is 15 times or more, preferably 17.5 times or more, and more preferably 20 times or more with respect to the median particle size of the active ingredient, the standard deviation of the active ingredient content within 5% or less, preferably 4% or less, and more preferably 3% or less. It is speculated that when the median particle size of excipients is large and the difference is large with respect to the active ingredient, composites can be formed with the active ingredient and excipients; moreover, by approximating the powder properties such as particle size, shape and bulk density of this composite to those of other additives, segregation between particles can be suppressed and the uniformity of the content of the active ingredient can be enhanced.

Content uniformity of the active ingredient in the drug product is generally assessed by the formulation uniformity test listed in the Japanese Pharmacopoeia. In addition, this evaluation is performed using values (judged values) calculated using the average content in addition to the standard deviation of individual content values. In the meantime, it was judged to be appropriate to evaluate only the degree of variance of the active ingredient using only the standard deviation of individual content values. When the standard deviation of the content value is 5% or less, it was generally considered preferable that the standard deviation of the active ingredient content is 5% or less because it meets the criteria of the formulation uniformity test.

When the active ingredient and excipient are mixed, and when the standard deviation of the active ingredient content in the drug product becomes more than 5%, it is possible to obtain a solid drug product in which the standard deviation of the active ingredient content in the drug product is 5% or less by mixing the excipient with the active ingredient using as the excipient that has a median particle size of 15 times or more with respect to the median particle size of the active ingredient. Also, by using an active ingredient with a median particle size of 15fold or less relative to the median particle size of the excipients, and by mixing the active ingredient with excipients, it is possible to obtain solid formulations with a standard deviation of within 5% of the active ingredient content in the formulation.

That is, the present invention is a useful technique in obtaining solid formulations in which the standard deviation of the active ingredient content in the formulation is 5% or less for an active ingredient such that the standard deviation of the active ingredient content in the formulation is more than 5% when the active ingredient and excipient are mixed.

In the present formulation, the lower limit of the median particle size of the excipients, which is also related to the median particle size with the active ingredient, is 30 µm or more, preferably 45 µm or more, and more preferably 60 µm or more. On the other hand, the upper limit of the median particle size of the excipients is 350 µm or less, preferably 300 µm or less, and more preferably 250 µm or less. When multiple types of excipients are used, the standard deviation of the active ingredient content is 5% or less, preferably 4% or less, and more preferably 3% or less, if the median particle size of excipients with the largest median particle size is 15-fold or more, preferably 17.5-fold or more, and more preferably 20-fold or more with respect to the median particle size of the active ingredient. When using multiple types of excipients, preferably, the excipients with the largest median particle size and the active ingredient are firstly mixed, followed by other excipients and additives. Even if the amount of excipients with the largest particle size is small, the active ingredient itself can be mixed with a sufficient quantity of excipients to make the standard deviation of the active ingredient content in the formulation within 5%, preferably if the active ingredient content in the formulation is less than or equal to 10 wt%, more preferably less than or equal to 5 wt%, especially preferably less than or equal to 1 wt%.

The formulation may contain a disintegrating agent. As disintegrating agents, those listed in the Japanese Pharmacopoeia, the Japanese Pharmaceutical Codex or the Japanese Pharmaceutical Excipients and the like can be used. Specifically, cross-carmellose sodium, cross-povidone, carmellose calcium, carboxymethyl starch sodium, low-substituted hydroxypropyl cellulose, and the like are enumerated, and preferably cross-carmellose sodium.

The content of disintegrating agent in the formulation is 0.5 to 30 wt%, preferably 0.75 to 25 wt%, and more preferably 1 to 20 wt%, relative to the total amount of the formulation.

This formulation may contain a lubricant, and it is possible to use a lubricant listed in the Japanese Pharmacopoeia, the Japanese Pharmaceutical Codex or the Japanese Pharmaceutical Excipients, and the like. Specific examples include metal stearate, sucrose fatty acid ester, talc, hydrous silicon dioxide, and the like, preferably include metal stearate. Metal stearate includes magnesium stearate, calcium stearate, and the like, preferably inclludes magnesium stearate.

The content of the lubricant is usually 0.05 to 10 wt%, preferably 0.075 to 7.5 wt%, and more preferably 0.1 to 5 wt%, relative to the total amount of the formulation.

The present formulation may contain a coating agent and the coating agent listed in the Japanese Pharmacopoeia, the Japanese Pharmaceutical Codex or the Japanese Pharmaceutical Excipients and the like can be used. Specifically, hypromellose (hydroxypropyl methylcellulose), polyvinyl alcohol, ethyl cellulose, carmellose, sodium carmellose, hydroxyethyl cellulose, hydroxyethyl methylcellulose, PVA copolymer, ethyl acrylate-methyl methacrylate copolymer dispersion, aminoalkyl methacrylate copolymer, opadly, carboxyvinyl polymer, dry methacrylate copolymer, dimethylaminoethyl methacrylate-methyl methacrylate copolymer, stearyl alcohol, shellac, setanol, hydroxypropyl methylcellulose acetate succinate, hydroxypropyl methylcellulose phthalate, and a mixture of fumaric acid, stearic acid, and polyvinyl acetal diethylaminoacetate and hydroxypropyl methylcellulose, polyvinyl acetal diethylaminoacetate, polyvinyl alcohol, methacrylic acid copolymer, 2-methyl-5-vinylpyridine methylacrylate methacrylate copolymer, and the like are included, preferably hypromellose (hydroxypropyl methylcellulose).

The content of the coating agent in the present formulation is usually 0.1 to 10 wt%, preferably 0.25 to 7.5 wt%, and more preferably 0.5 to 5 wt%, relative to the total amount of the formulation.

Plasticizers and anti-coagulants may be included in the coating agent to efficiently perform the coating operation, and those listed in the Japanese Pharmacopoeia, the Japanese Pharmaceutical Codex, the Japanese Pharmaceutical Excipients or Japan's specifications and standards for food additives and the like can be used. Specific examples include macrogol 1000, macrogol 1500, macrogol 1540, macrogol 4000, macrogol 6000, macrogol 8000, macrogol 20000, macrogol 35000, and the like (polyethylene glycol with an average molecular weight of 1000 to 35000), glycerin fatty acid esters, sucrose fatty acid esters, castor oil, talc, and the like.

The formulation may contain dyes or colorants, and dyes listed in the Japanese Pharmacopoeia, the Japanese Pharmaceutical Codex or the Japanese Pharmaceutical Excipients and the like can be used. Dyes can be contained both in tablets and in coating layers. Dyes specifically include iron oxide, tar dyes and natural dyes. Iron oxides include iron tridioxide, iron yellow oxide, yellow iron trioxide, black iron oxide, and the like. Tar dyes include edible yellow No. 4 aluminum lakes, edible blue No. 1 aluminum lakes, edible red No. 3 aluminum lakes, edible blue No. 1, edible blue No. 2, edible yellow No. 4, edible yellow No. 5, edible red No. 102, edible red No. 2, edible red No. 3, etc. Natural pigments include turmeric extract, β-carotene, carotene solution, copper chlorophyllin sodium, copper chlorophyll, powdered green leaf extract of red wheat, dry powder of bare green leaf blue juice, and extract of bare wheat green leaf.

The formulation may further contain additives other than those listed above, if necessary, and additives listed in the Japanese Pharmacopoeia, the Japanese Pharmaceutical Codex, the Japanese Pharmaceutical Excipients or Japan's specifications and standards for food additives can be used. Also, the content of these additives may be at any rate. Additive agents other than those described above specifically include binders, flavors, fluidizers, flavoring agents, flavoring agents, and the like.

Specific examples of the binder include hydroxypropyl cellulose, corn starch, alphalized starch, portion alphalized starch, gum arabic, gum arabic powder, gelatin, agar, dextrin, pullulan, polyvinylpyrrolidone, polyvinyl alcohol, crystalline cellulose, methylcellulose, ethyl cellulose, carboxymethyl ethyl cellulose, carmelose, sodium carmelose, hydroxy ethyl cellulose, hydroxy ethyl methylcellulose, hydroxy propyl cellulose, hypromellose, and the like.

Flavoring agents include orange essence, orange oil, caramel, camphor, silkworm oil, spearmint oil, stroberry essence, chocolate essence, cherry flavor, spruce oil, pine oil, hacka oil, vanilla flavor, bitter essence, fruit flavor, peppermint essence, mix flavor, mint flavor, menthol, lemon powder, lemon oil, rose oil, and the like.

Fluidizing agents specifically include hydrous silicon dioxide, light silicic anhydride, crystalline cellulose, synthetic aluminum silicate, talc, and the like.

Taste correction agents specifically include aspartame, sucralose, glycine, sodium chloride, magnesium chloride, hydrochloric acid, dilute hydrochloric acid, citric acid and its salts, citric anhydrous, L-glutamic acid and its salts, succinic acid and its salts, acetic acid, tartaric acid and its salts, sodium bicarbonate, fumaric acid and its salts, malic acid and its salts, glacial acetic acid, disodium inosinate, honey, and the like.

The present formulation is a solid formulation. Specifically, granules, fine granules, tablets, powders, capsules, pills, etc. may be used, preferably granules or tablets.

The manufacturing method of granules of the present formulation is not particularly limited as long as it is a manufacturing method with good content uniformity of the active ingredient and no decomposition of the active ingredient, but specifically, it is a method of mixing additives such as active ingredient, disintegrating agent, excipient, etc. to granulate the mixed powder after the production, and it is a dry granulation method, fracture granulation method, and melt granulation method, preferably by compression forming without using water. The granules can also be coated by coating the granules with a coating agent after the granules are made. V-type mixing machines and container blenders can be used as machines to mix active ingredients, additives, etc. Also, dry fracturing granulating, fracturing granulators, and melt extrusion granulators can be used as the granulating machines.

The manufacturing method of the tablet of the present formulation is not particularly limited as long as the formulation method has good content uniformity of the active ingredient and does not degrade the active ingredient, but specifically, it is a direct compression method in which an additive such as an active ingredient, a disintegrating agent, or an excipient is mixed to produce a mixed powder, and then the mixed powder is tableted by a tablet-pressor. V-type mixing machines and container blenders can be used as machines to mix active ingredients, additives, etc. Further, as the compression machine, a single compression machine, a rotary compression machine, or the like can be used.

If the manufacturing method of the present formulation is a manufacturing method characterized by including the process of mixing the excipient having a median particle size of 10 µm or less of the active ingredient and a median particle size of 30 µm or more of the excipient in the formulation and a median particle size of 15 times or more with respect to the median particle size of the active ingredient, the standard deviation of the active ingredient content in the formulation can be 5% or less.

If the manufacturing method of the present formulation is a manufacturing method characterized by including the process of mixing the excipient having a median particle size of 8 µm or less of the active ingredient and a median particle size of 45 µm or more of the excipient in the formulation and a median particle size of 17.5 times or more with respect to the median particle size of the active ingredient, the standard deviation of the active ingredient content in the formulation can be 5% or less.

If the manufacturing method of the present formulation is a manufacturing method characterized by including the process of mixing the excipient having a median particle size of 5 µm or less of the active ingredient and a median particle size of 60 µm or more of the excipient in the formulation and a median particle size of 20 times or more with respect to the median particle size of the active ingredient, the standard deviation of the active ingredient content in the formulation can be 5% or less.

If the manufacturing method of the present formulation, wherein the active ingredient content can be 10wt% or less, is a manufacturing method characterized by including the process of mixing the excipient having a median particle size of 10 µm or less of the active ingredient and a median particle size of 30 µm or more of the excipient in the formulation and a median particle size of 15 times or more with respect to the median particle size of the active ingredient, the standard deviation of the active ingredient content in the formulation can be 5% or less.

If the manufacturing method of the present formulation, wherein the active ingredient content can be 5wt% or less, is a manufacturing method characterized by including the process of mixing the excipient having a median particle size of 8 µm or less of the active ingredient and a median particle size of 45 µm or more of the excipient in the formulation and a median particle size of 17.5 times or more with respect to the median particle size of the active ingredient, the standard deviation of the active ingredient content in the formulation can be 4% or less.

If the manufacturing method of the present formulation, wherein the active ingredient content can be 1wt% or less, is a manufacturing method characterized by including the process of mixing the excipient having a median particle size of 5 µm or less of the active ingredient and a median particle size of 60 µm or more of the excipient in the formulation and a median particle size of 20 times or more with respect to the median particle size of the active ingredient, the standard deviation of the active ingredient content in the formulation can be 3% or less.

If the manufacturing method of the present formulation, wherein the active ingredient content can be 10wt% or less in the formulation, is a manufacturing method characterized by including the process of mixing the excipient having a median particle size of 10 µm or less of the active ingredient and a median particle size of 30 µm or more of the excipient in the formulation and (i) the process of mixing the active ingredient, and the excipient of a median particle size of 20 times or more with respect to the median particle size of the active ingredient, (ii) the process of compressively forming the mixture by direct compression or dry granulation method, the standard deviation of the active ingredient content in the formulation can be 5% or less.

If the manufacturing method of the present formulation, wherein the active ingredient content can be 5wt% or less in the formulation , is a manufacturing method characterized by including the process of mixing the excipient having a median particle size of 8 µm or less of the active ingredient and a median particle size of 45 µm or more of the excipient in the formulation and (i) the process of mixing the active ingredient, and the excipient of a median particle size of 17.5 times or more with respect to the median particle size of the active ingredient, (ii) the process of compressively forming the mixture by direct compression or dry granulation method, the standard deviation of the active ingredient content in the formulation can be 4% or less.

If the manufacturing method of the present formulation, wherein the active ingredient content can be 1wt% or less in the formulation, is a manufacturing method characterized by including the process of mixing the excipient having a median particle size of 5 µm or less of the active ingredient and a median particle size of 60 µm or more of the excipient in the formulation and (i) the process of mixing the active ingredient, and the excipient of a median particle size of 20 times or more with respect to the median particle size of the active ingredient, (ii) the process of compressively forming the mixture by direct compression or dry granulation method, the standard deviation of the active ingredient content in the formulation can be 3% or less.

The manufacturing method of the present formulation allows the production of solid formulations with standard deviations of 5% or less and preferably 3% or less of the active ingredient content in the formulation.

The prescriptions of present formulations are specifically solid formulations containing the water-labile compound represented by formula (IA), its pharmaceutically acceptable salts or its solvates as active ingredients and excipients, with an active ingredient content of 10 wt % or less and a standard deviation of the active ingredient content within 5 %, preferably solid formulations containing the compound of formula (IA) or its solvates as active ingredients and excipients, with an active ingredient content of 5 wt % or less and a standard deviation of the active ingredient content within 4 %, and more preferably solid formulations containing the compound of formula (IA) or its solvates as active ingredients and excipients, with an active ingredient content of 1 wt % or less and a standard deviation of the active ingredient content within 3 %.

The prescriptions of present formulations are specifically solid formulations containing the water-labile compound of formula (IA), its pharmaceutically acceptable salts or its solvates as active ingredients, and 50 to 99.99 wt% excipients, with an active ingredient content of 10 wt % or less and a standard deviation of the active ingredient content within 5 %, preferably solid formulations containing the compound of formula (IA) or its solvates as active ingredients and 60 to 99.99 wt% excipients, with an active ingredient content of 5 wt % or less and a standard deviation of the active ingredient content within 4 %, and more preferably solid formulations containing the compound of formula (IA) or its solvates as active ingredients and 70 to 99.99 wt% excipients, with an active ingredient content of 1 wt % or less and a standard deviation of the active ingredient content within 3 %.

The prescriptions of present formulations are specifically solid formulations containing the water-labile compound of formula (IA), its pharmaceutically acceptable salts or its solvates as active ingredients, and excipients, with an active ingredient content of 10 wt % or less and a standard deviation of the active ingredient content within 5 %, preferably solid formulations containing the compound of formula (IA) or its solvates as active ingredients and excipients, with an active ingredient content of 5 wt % or less and a standard deviation of the active ingredient content within 4 %, and more preferably solid formulations containing the compound of formula (IA) or its solvates as active ingredients and excipients, with an active ingredient content of 1 wt % or less and a standard deviation of the active ingredient content within 3 %.

The prescriptions of present formulations are specifically solid formulations containing the water-labile compound of formula (IA), its pharmaceutically acceptable salts or its solvates as active ingredients, and 50 to 99.99 wt% excipients, with an active ingredient content of 10 wt % or less and a standard deviation of the active ingredient content within 5 %, preferably solid formulations containing the compound of formula (IA) or its solvates as active ingredients and 60 to 99.99 wt% excipients, with an active ingredient content of 5 wt % or less and a standard deviation of the active ingredient content within 4 %, and more preferably solid formulations containing the compound of formula (IA) or its solvates as active ingredients and 70 to 99.99 wt% excipients, with an active ingredient content of 1 wt % or less and a standard deviation of the active ingredient content within 3 %.

The prescriptions of present formulations are specifically solid formulations containing the salts of p-toluenesulfonic acid salt, or a solvate thereof of the compound represented by the formula(IA) as the active ingredient and excipients, and the active ingredient content is 10 wt.% or less, and a standard deviation of the active ingredient content within 5 %, preferably solid formulations containing the salts of p-toluenesulfonic acid salt, or a solvate thereof of the compound represented by the formula(IA) as the active ingredient and excipients, and the active ingredient content is 5 wt.% or less, and the standard deviation of the active ingredient content within 4%, more preferably solid formulations containing the salts of p-toluenesulfonic acid salt, or a solvate thereof of the compound represented by the formula(IA) as the active ingredient and excipients, and the active ingredient content is 1 wt.% or less, and the standard deviation of the active ingredient content within 3%.

The prescriptions of present formulations are specifically solid formulations containing the salts of p-toluenesulfonic acid salt, or a solvate thereof of the compound represented by the formula(IA) as the active ingredient and 50-99.99 wt% excipients, and the active ingredient content is 10 wt.% or less, and a standard deviation of the active ingredient content within 5 %, preferably solid formulations containing the salts of p-toluenesulfonic acid salt, or a solvate thereof of the compound represented by the formula(IA) as the active ingredient and 60-99.99 wt% excipients, and the active ingredient content is 5 wt.% or less, and the standard deviation of the active ingredient content within 4%, more preferably solid formulations containing the salts of p-toluenesulfonic acid salt, or a solvate thereof of the compound represented by the formula(IA) as the active ingredient and 70-99.99 wt% excipients, and the active ingredient content is 1 wt.% or less, and the standard deviation of the active ingredient content within 3%.

As the tablet shape, any shape can be adopted, and specifically, it can be a tablet with a round, oval, spherical, rod-shaped, or doughnut-shaped shape. It may also be a stacked tablet, a nucleated tablet, etc., preferably a single-layer tablet with a convenient manufacturing method is preferable. Furthermore, marks, letters, and the like may be inscribed to improve the discrimination, as well as allocation lines for division.

### EXAMPLES:

The present invention will be explained in more detail below by way of Examples, Comparative Examples and Reference Examples, but these do not limit the present invention.
(1) Effect of median particle size of active ingredients and excipients on content uniformity.

The effect of the median particle size of the active ingredients and excipients on the content uniformity of the active ingredients was studied. Content uniformity represents whether the active ingredient is contained without segregation in formulations such as granules and tablets. As active ingredients, p-toluenesulfonic acid salt of compounds of the formula (IA) and D-mannitol (made by Roquette or Merck) were used as excipients. After mixing powders formulated with active ingredients and excipients of various median particle sizes in Table 1 in wt.% of Table 1, the active ingredient content of the compacted tablets was determined by the following method and their standard deviations were determined from the results:

The tablets of Examples 1 and 2 and Comparative Example 1 were prepared under the following conditions: 3.906 g p-toluenesulfonic acid salt (3 g as a compound of the formula (IA)) of the compound of the formula (IA) and 2366 g D-mannitol, 600 g of low-substituted hydroxypropyl cellulose, are sieved three times with a wire mesh of 30 mesh followed by mixing at 37 rpm for 33 min using a V-type mixing machine (effective volume 8 L). To the mixture add 30 g of magnesium stearate sieved through a 30-mesh wire mesh and mix at 37 rpm for 5 minutes. The resulting mixtures were also compacted to produce tablets using a rotary compression machine (Kikuchi Manufacturing RTM-S30K-2 S type).

The tablets used in Example 3 were produced under the following conditions: p-toluenesulfonic acid salt 234. 4 g of the compound of the formula (IA) (180 g as compound of the formula (IA)) and 96,430 g of D-mannitol, 1,080 g of sodium cross-carmellose are sieved once with a 30-mesh wire mesh followed by mixing at 20 rpm for 27 min using a V-type mixing machine (effective volume 273 L). To the mixture add 540 g of magnesium stearate sieved through a 30-mesh wire mesh and mix at 20 rpm for 2.7 minutes. The resulting mixtures were also compacted to produce tablets using a rotary compression machine (Kikuchi Manufacturing LIBRA2).

The median particle size (volume-averaged particle size) of the active ingredient was determined using a particle size measuring machine made by HELOS(H1086) & ROSOS (manufactured by Nippon Laser) with a focal length of 100 mm and a dispersive pressure of 2 bars under the following conditions:

### HELOS (Laser diffraction unit)

| | |
|---|---|
| Type | : HELOS(H1086) |
| Lens | : R3 |
| Measuring range | : 0.5-175µm |
| Trigger condition | : 2s-100ms-k15-0.5%-0.2% |
| Calculation mode | : HRLD (5.3.0.0) |
| RODOS (Dispersing system) | |
| Feeder | : VIBRI |
| Dispersion pressure | : 2.00bar |
| Degree of vacuum | : 100mbar |
| Feeding | : 50.00% |
| Rotation | : 30.00% |
| Density | : 1.00g/cm³ |
| Shape counting | : 1.00 |

In addition, the median particle size (volume-averaged particle size) of the excipients was evaluated under the conditions of a focal length of 100 mm and a dispersive pressure of 3 bar using a particle size measuring machine of HELOS(H1086)&ROSOS (manufactured by Nippon Laser).

### HELOS (Laser diffraction unit)

| | |
|---|---|
| Type | : HELOS(H1086) |
| Lens | : R5 |
| Measuring range | : 0.5-875µm |
| Trigger condition | : 2s-100ms-conc-1.0%-1.0% |
| Calculation mode | : HRLD (5.8.0.0) |
| RODOS (Dispersing system) | |
| Feeder | : VIBRI |
| Dispersion pressure | : 3.00bar |
| Degree of vacuum | : 134mbar |
| Feeding | : 45.00% |
| Rotation | : 20.00% |
| Density | : 1.00g/cm³ |
| Shape counting | : 1.00 |

### (Determination of the standard deviation of the content)

In Examples 1, 2 and Comparative Example 1, samples were taken out of 10 tablets each for a total of 40 tablets during the compression process at regular intervals for a total of 4 times, including at the start and end of compression, and their contents were determined by the test method described below.

In Example 3, samples were drawn from three tablets and 75 tablets in total at regular intervals for a total of 25 tablets during the compression process, including at the start and end of compression, respectively, and the contents of these tablets were measured by the test method described below.

The method for determination of the content of the formula (IA) compounds of the active ingredients was determined by the HPLC method (wavelength: 240nm, column: L-column ODS (filler 5µm, 4.6×250mm, manufactured by the Chemical Assessment and Research Organization), column temperature: 45°C, mobile phase: a mixture of 20mmol/L phosphate buffer solution/acetonitrile (13:7) in pH5. 5, flow rate: 1.0mL/min.

### (Result)

**[Table 1]**

| | | Example 1 | Example 2 | Example 3 | Comparative Example 1 |
|---|---|---|---|---|---|
| Content in the formulation (wt%) | active ingredient* | 0.13 | 0.13 | 0.22 | 0.13 |
| | D-mannitol | 78.87 | 78.87 | 89.28 | 78.87 |
| | Low substituted hydroxypropyl cellulose | 20.0 | 20.0 | - | 20.0 |
| | Croscarmellose sodium | - | - | 10.0 | - |
| | Magnesium stearate | 1.0 | 1.0 | 0.5 | 1.0 |
| | Total | 100.0 | 100.0 | 100.0 | 100.0 |
| Median particle size(µm) | D- mannitol | 91.9 | 91.9 | 62.5 | 91.9 |
| | Active ingredient | 4.3 | 3.0 | 3.3 | 9.9 |
| D-mannitol/active ingredient particle size ratio | | 21.3 | 30.6 | 18.9 | 9.3 |
| Content of the active ingredient (%) | Average | 99.7 | 99.7 | 98.8 | 99.1 |
| | Standard deviation | 1.9 | 1.2 | 0.8 | 6.1 |

| | | | | | |
|---|---|---|---|---|---|
| *: Described as the amount of p-toluenesulfonic acid salt of the formula (IA) compound | | | | | |

In Example 3, wherein the ratio of the median particle size of the active ingredient and D-mannitol was 18.9, the standard deviations of the active ingredient contents were 0.8% and the standard deviation was 5% or less. The ratio of the median particle size of the active ingredient and D-mannitol in Examples 1 and 2 was greater than 18.9 in Example 3, although the standard deviation of the content was less than 5% for both formulations. On the other hand, the ratio of the median particle size of the active ingredient and D-mannitol in Comparative Example 1 was less than 10, the standard deviation of the content became greater than 5%, and the content uniformity was low. Therefore, it was revealed that the standard deviation of the content became 5% or less, when the median particle size of the excipient with respect to the active ingredient was 15 times or more.

### Industrial applicability

By mixing the active ingredient, and excipients with a median particle size of 15 times or more, preferably 20 times or more, relative to the median particle size of the active ingredient, solid formulations with good content uniformity can be produced, e.g., tablets, granules, powders and capsules. Since the manufacturing method of the present formulation is not cumbersome, the working efficiency of the formulation production is also high.

## Claims

1. A process for manufacturing a solid formulation **characterized in** including a step of mixing an active ingredient and an excipient which has a median particle size of 15 times or more to the median particle size of the said active ingredient, wherein the standard deviation of content in the active ingredient is 5% or less, wherein the active ingredient is a compound represented by the formula (IA): 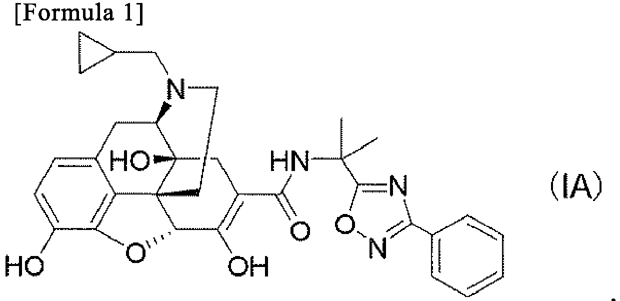 its pharmaceutically acceptable salt, or a solvate thereof.

2. The process for manufacturing a solid formulation according to claim 1, **characterized by** including the following steps:
(i) a step of mixing the active ingredient, and the excipient which has the median particle size of 15 times or more to the median particle size of the said active ingredient,
(ii) a step of compression molding the said mixture by the method of direct compression or dry granulation.

3. The process for manufacturing a solid formulation according to claim 1 or 2, wherein the median particle size of the excipient is 20 times or more to the median particle size of the active ingredient.

4. The process for manufacturing a solid formulation according to any one of claims 1 to 3, wherein the standard deviation of content on the active ingredient is 3% or less in the formulation.

5. The process for manufacturing a solid formulation according to any one of claims 1 to 4, wherein the content of the active ingredient is 10% or less by weight in the formulation.

6. The process for manufacturing a solid formulation according to claim 5, wherein the content of the active ingredient is 5% or less by weight in the formulation.

7. The process for manufacturing of a solid formulation according to claim 5, wherein the content of the active ingredient is 1% or less by weight in the formulation.

8. The process for manufacturing of a solid formulation according to any one of claims 1 to 7, wherein the content of the excipient is 50 to 99.99% by weight in the formulation.

9. The process for manufacturing a solid formulation according to claim 8, wherein the content of an excipient is 60 to 99.99% by weight in the formulation.

10. The process for manufacturing a solid formulation according to claim 8, wherein the content of an excipient is 70 to 99.99% by weight in the formulation.

11. The process for manufacturing a solid formulation according to any one of claims 1 to 10, wherein the median particle size of the active ingredient is 10 µm or less.

12. The process for manufacturing a solid formulation according to any one of claims 1 to 10, wherein the median particle size of the active ingredient is 8 µm or less.

13. The process for manufacturing a solid formulation according to claim 11, wherein the median particle size of the active ingredient is 5 µm or less.

14. The process for manufacturing a solid formulation according to any one of claims 1 to 13, wherein the median particle size of the excipient is 30 µm or more.

15. The process for manufacturing a solid formulation according to claim 14, wherein the median particle size of the excipient is 45 µm or more.

16. The process for manufacturing a solid formulation according to claim 14, wherein the median particle size of the excipient is 60 µm or more.

17. The process for manufacturing a solid formulation according to claim 11 or 14, wherein the median particle size of the active ingredient is 10 µm or less and the median particle size of the excipient is 30 µm or more.

18. The process for manufacturing a solid formulation according to any one of claims 1 to 17, wherein the content of the active ingredient in the formulation is 1% or less, the median particle size of the said active ingredient is 5 µm or less, the median particle size of the excipient is 60 µm or more, and **characterized in** including the step of mixing the active ingredient and the excipient which has the median particle size of 20 times or more to the median particle size of the said active ingredient, wherein the standard deviation of content in the active ingredient is 3% or less in the formulation.

19. The process for manufacturing a solid formulation according to claim 18, wherein the content of the active ingredient in the formulation is 1 wt % or less, the median particle size of said active ingredient is 5 µm or less, the median particle size of the excipient is 60µm or more, and **characterized in** including (i) the step of mixing the active ingredient and the excipient which has the median particle size of 20 times to the median particle size of the said active ingredient, (ii) the step of compression molding the said mixture by the method of direct compression or dry granulation.

20. The process for manufacturing a solid formulation according to claim 1, containing p-toluene sulfonic acid salt, acetate or hydrochloride salt of the compound represented by the formula (IA), or a solvate of the salt as the active ingredient.

21. The process for manufacturing a solid formulation according to any one of claims 1 to 20, containing sugar or sugar alcohol as the excipient.

22. The process for manufacturing a solid formulation according to claim 21, containing D-mannitol as the excipient.

23. The process for manufacturing a solid formulation according to any one of claims 1 to 22, wherein the solid formulation is one or more selected from the group consisting of tablet, granule, powder and capsule.

24. A solid formulation produced by the process for manufacturing, according to any one of claims 1 to 23.

25. The solid formulation according to claim 24, containing an unstable compound, its pharmaceutically acceptable salt or its solvate as an active ingredient to water and a excipient, wherein the content of the active ingredient is 1% or less and the standard deviation of content on the active ingredient is 3% or less in the formulation, wherein the active ingredient is a compound represented by the formula (IA): 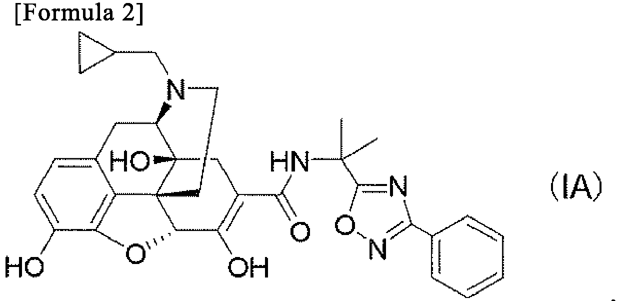 its pharmaceutically acceptable salt, or a solvate thereof.

26. The solid formulation according to claim 25, wherein the content of the excipient is 70 to 99.99 % by weight in the formulation.

## Patentansprüche

1. Ein Verfahren zur Herstellung einer festen Formulierung, **dadurch gekennzeichnet, dass** es einen Schritt des Mischens eines Wirkstoffs und eines Exzipienten beinhaltet, dessen mittlere Teilchengröße das 15-Fache oder mehr der mittleren Teilchengröße des Wirkstoffs beträgt, wobei die Standardabweichung des Gehalts an Wirkstoff 5 % oder weniger beträgt, wobei der Wirkstoff eine Verbindung der Formel (IA) darstellt: 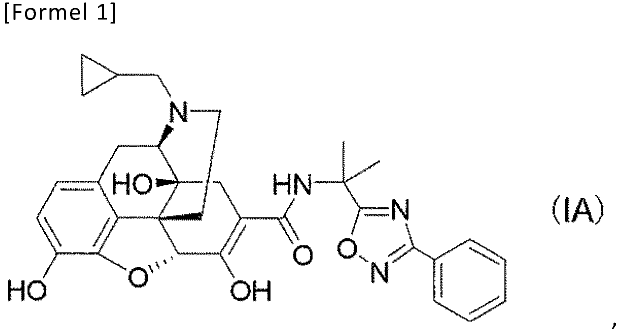 ihr pharmazeutisch verträgliches Salz oder ein Solvat davon.

2. Das Verfahren zur Herstellung einer festen Formulierung gemäß Anspruch 1, **dadurch gekennzeichnet, dass** es die folgenden Schritte beinhaltet:
(i) einen Schritt des Mischens des Wirkstoffs und des Exzipienten, dessen mittlere Teilchengröße das 15-Fache oder mehr der mittleren Teilchengröße des Wirkstoffs beträgt,
(ii) einen Schritt des Formpressens des Gemischs durch das Verfahren der Direktverpressung oder der Trockengranulierung.

3. Das Verfahren zur Herstellung einer festen Formulierung gemäß Anspruch 1 oder 2, wobei die mittlere Teilchengröße des Exzipienten das 20-Fache oder mehr der mittleren Teilchengröße des Wirkstoffs beträgt.

4. Das Verfahren zur Herstellung einer festen Formulierung gemäß einem der Ansprüche 1 bis 3, wobei die Standardabweichung des Gehalts an Wirkstoff in der Formulierung 3 % oder weniger beträgt.

5. Das Verfahren zur Herstellung einer festen Formulierung gemäß einem der Ansprüche 1 bis 4, wobei der Gehalt des Wirkstoffs in der Formulierung 10 Gew.-% oder weniger beträgt.

6. Das Verfahren zur Herstellung einer festen Formulierung gemäß Anspruch 5, wobei der Gehalt des Wirkstoffs in der Formulierung 5 Gew.-% oder weniger beträgt.

7. Das Verfahren zur Herstellung einer festen Formulierung gemäß Anspruch 5, wobei der Gehalt des Wirkstoffs in der Formulierung 1 Gew.-% oder weniger beträgt.

8. Das Verfahren zur Herstellung einer festen Formulierung gemäß einem der Ansprüche 1 bis 7, wobei der Gehalt des Exzipienten in der Formulierung 50 bis 99,99 Gew.-% beträgt.

9. Das Verfahren zur Herstellung einer festen Formulierung gemäß Anspruch 8, wobei der Gehalt des Exzipienten in der Formulierung 60 bis 99,99 Gew.-% beträgt.

10. Das Verfahren zur Herstellung einer festen Formulierung gemäß Anspruch 8, wobei der Gehalt des Exzipienten in der Formulierung 70 bis 99,99 Gew.-% beträgt.

11. Das Verfahren zur Herstellung einer festen Formulierung gemäß einem der Ansprüche 1 bis 10, wobei die mittlere Teilchengröße des Wirkstoffs 10 µm oder weniger beträgt.

12. Das Verfahren zur Herstellung einer festen Formulierung gemäß einem der Ansprüche 1 bis 10, wobei die mittlere Teilchengröße des Wirkstoffs 8 µm oder weniger beträgt.

13. Das Verfahren zur Herstellung einer festen Formulierung gemäß Anspruch 11, wobei die mittlere Teilchengröße des Wirkstoffs 5 µm oder weniger beträgt.

14. Das Verfahren zur Herstellung einer festen Formulierung gemäß einem der Ansprüche 1 bis 13, wobei die mittlere Teilchengröße des Exzipienten 30 µm oder mehr beträgt.

15. Das Verfahren zur Herstellung einer festen Formulierung gemäß Anspruch 14, wobei die mittlere Teilchengröße des Exzipienten 45 µm oder mehr beträgt.

16. Das Verfahren zur Herstellung einer festen Formulierung gemäß Anspruch 14, wobei die mittlere Teilchengröße des Exzipienten 60 µm oder mehr beträgt.

17. Das Verfahren zur Herstellung einer festen Formulierung gemäß Anspruch 11 oder 14, wobei die mittlere Teilchengröße des Wirkstoffs 10 µm oder weniger und die mittlere Teilchengröße des Exzipienten 30 µm oder mehr beträgt.

18. Das Verfahren zur Herstellung einer festen Formulierung gemäß einem der Ansprüche 1 bis 17, wobei der Gehalt des Wirkstoffs in der Formulierung 1 % oder weniger beträgt, die mittlere Teilchengröße des Wirkstoffs 5 µm oder weniger beträgt, die mittlere Teilchengröße des Exzipienten 60 µm oder mehr beträgt, und **dadurch gekennzeichnet ist, dass** es den Schritt des Mischens des Wirkstoffs und des Exzipienten beinhaltet, dessen mittlere Teilchengröße das 20-Fache oder mehr der mittleren Teilchengröße des Wirkstoffs beträgt, wobei die Standardabweichung des Gehalts an Wirkstoff in der Formulierung 3 % oder weniger beträgt.

19. Das Verfahren zur Herstellung einer festen Formulierung gemäß Anspruch 18, wobei der Gehalt des Wirkstoffs in der Formulierung 1 Gew.-% oder weniger beträgt, die mittlere Teilchengröße des Wirkstoffs 5 µm oder weniger beträgt, die mittlere Teilchengröße des Exzipienten 60 µm oder mehr beträgt, und **dadurch gekennzeichnet ist, dass** es (i) den Schritt des Mischens des Wirkstoffs und des Exzipienten, dessen mittlere Teilchengröße das 20-Fache der mittleren Teilchengröße des Wirkstoffs beträgt, (ii) den Schritt des Formpressens des Gemischs durch das Verfahren der Direktverpressung oder Trockengranulierung beinhaltet.

20. Das Verfahren zur Herstellung einer festen Formulierung gemäß Anspruch 1, welche ein p-Toluolsulfonsäuresalz, ein Acetat oder ein Hydrochloridsalz der gemäß der Formel (IA) dargestellten Verbindung oder ein Solvat des Salzes als den Wirkstoff enthält.

21. Das Verfahren zur Herstellung einer festen Formulierung gemäß einem der Ansprüche 1 bis 20, welche Zucker oder Zuckeralkohol als den Exzipienten enthält.

22. Das Verfahren zur Herstellung einer festen Formulierung gemäß Anspruch 21, welche D-Mannitol als den Exzipienten enthält.

23. Das Verfahren zur Herstellung einer festen Formulierung gemäß einem der Ansprüche 1 bis 22, wobei die feste Formulierung eine oder mehrere, ausgewählt aus der Gruppe bestehend aus Tablette, Granulat, Pulver und Kapsel, ist.

24. Eine feste Formulierung, welche durch das Herstellungsverfahren gemäß einem der Ansprüche 1 bis 23 hergestellt wurde.

25. Die feste Formulierung gemäß Anspruch 24, enthaltend eine gegenüber Wasser instabile Verbindung, ihr pharmazeutisch verträgliches Salz oder Solvat davon als einen Wirkstoff, und einen Exzipienten, wobei der Gehalt des Wirkstoffs 1 % oder weniger beträgt und die Standardabweichung des Gehalts an Wirkstoff in der Formulierung 3 % oder weniger beträgt, wobei der Wirkstoff eine Verbindung dargestellt durch die Formel (IA) ist: 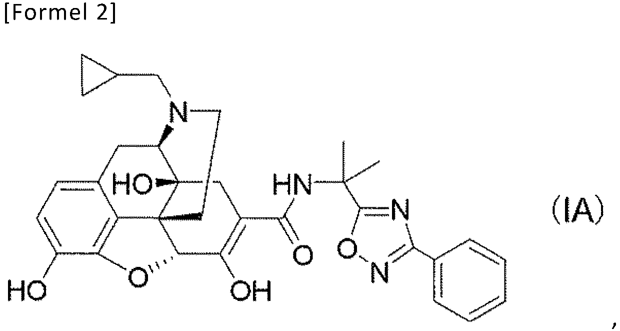 ihr pharmazeutisch verträgliches Salz oder ein Solvat davon.

26. Die feste Formulierung gemäß Anspruch 25, wobei der Gehalt des Exzipienten in der Formulierung 70 bis 99,99 Gew.-% beträgt.

## Revendications

1. Procédé pour fabriquer une formulation solide, **caractérisé en ce qu'**il comprend une étape de mélange d'un principe actif et d'un excipient qui a une taille médiane de particules de 15 fois ou plus la taille médiane de particules dudit principe actif, dans lequel l'écart type de la teneur en le principe actif est de 5 % ou moins, dans lequel le principe actif est un composé représenté par la formule (IA) : 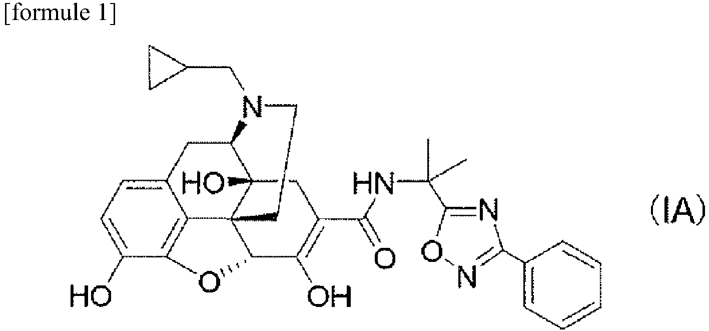 son sel pharmaceutiquement acceptable, ou un solvate de celui-ci.

2. Procédé pour fabriquer une formulation solide selon la revendication 1, **caractérisé en ce qu'**il comprend les étapes suivantes :
(i) une étape de mélange du principe actif et de l'excipient qui a une taille médiane de particules de 15 fois ou plus la taille médiane de particules dudit principe actif,
(ii) une étape de moulage par compression dudit mélange par la méthode de compression directe ou de granulation à sec.

3. Procédé pour fabriquer une formulation solide selon la revendication 1 ou 2, dans lequel la taille médiane de particules de l'excipient est de 20 fois ou plus la taille médiane de particules du principe actif.

4. Procédé pour fabriquer une formulation solide selon l'une quelconque des revendications 1 à 3, dans lequel l'écart type de la teneur en le principe actif est de 3 % ou moins dans la formulation.

5. Procédé pour fabriquer une formulation solide selon l'une quelconque des revendications 1 à 4, dans lequel la teneur en le principe actif est de 10 % en poids ou moins dans la formulation.

6. Procédé pour fabriquer une formulation solide selon la revendication 5, dans lequel la teneur en le principe actif est de 5 % en poids ou moins dans la formulation.

7. Procédé pour fabriquer une formulation solide selon la revendication 5, dans lequel la teneur en le principe actif est de 1 % en poids ou moins dans la formulation.

8. Procédé pour fabriquer une formulation solide selon l'une quelconque des revendications 1 à 7, dans lequel la teneur en l'excipient est de 50 à 99,99 % en poids dans la formulation.

9. Procédé pour fabriquer une formulation solide selon la revendication 8, dans lequel la teneur en l'excipient est de 60 à 99,99 % en poids dans la formulation.

10. Procédé pour fabriquer une formulation solide selon la revendication 8, dans lequel la teneur en l'excipient est de 70 à 99,99 % en poids dans la formulation.

11. Procédé pour fabriquer une formulation solide selon l'une quelconque des revendications 1 à 10, dans lequel la taille médiane de particules du principe actif est de 10 µm ou moins.

12. Procédé pour fabriquer une formulation solide selon l'une quelconque des revendications 1 à 10, dans lequel la taille médiane de particules du principe actif est de 8 µm ou moins.

13. Procédé pour fabriquer une formulation solide selon la revendication 11, dans lequel la taille médiane de particules du principe actif est de 5 µm ou moins.

14. Procédé pour fabriquer une formulation solide selon l'une quelconque des revendications 1 à 13, dans lequel la taille médiane de particules de l'excipient est de 30 µm ou plus.

15. Procédé pour fabriquer une formulation solide selon la revendication 14, dans lequel la taille médiane de particules de l'excipient est de 45 µm ou plus.

16. Procédé pour fabriquer une formulation solide selon la revendication 14, dans lequel la taille médiane de particules de l'excipient est de 60 µm ou plus.

17. Procédé pour fabriquer une formulation solide selon la revendication 11 ou 14, dans lequel la taille médiane de particules du principe actif est de 10 µm ou moins et la taille médiane de particules de l'excipient est de 30 µm ou plus.

18. Procédé pour fabriquer une formulation solide selon l'une quelconque des revendications 1 à 17, dans lequel la teneur en le principe actif dans la formulation est de 1 % ou moins, la taille médiane de particules dudit principe actif est de 5 µm ou moins, la taille médiane de particules de l'excipient est de 60 µm ou plus, et **caractérisé en ce qu'**il comprend une étape de mélange du principe actif et de l'excipient qui a une taille médiane de particules de 20 fois ou plus la taille médiane de particules dudit principe actif, dans lequel l'écart type de la teneur en le principe actif est de 3 % ou moins dans la formulation.

19. Procédé pour fabriquer une formulation solide selon la revendication 18, dans lequel la teneur en le principe actif dans la formulation est de 1 % en poids ou moins, la taille médiane de particules dudit principe actif est de 5 µm ou moins, la taille médiane de particules de l'excipient est de 60 µm ou plus, et **caractérisé en ce qu'**il comprend (i) l'étape de mélange du principe actif et de l'excipient qui a une taille médiane de particules de 20 fois la taille médiane de particules dudit principe actif, (ii) l'étape de moulage par compression dudit mélange par la méthode de compression directe ou de granulation à sec.

20. Procédé pour fabriquer une formulation solide selon la revendication 1, contenant un sel d'acide p-toluènesulfonique, un sel chlorhydrate ou acétate du composé représenté par la formule (IA), ou un solvate du sel, en tant que principe actif.

21. Procédé pour fabriquer une formulation solide selon l'une quelconque des revendications 1 à 20, contenant un sucre ou un alcool de sucre, en tant qu'excipient.

22. Procédé pour fabriquer une formulation solide selon la revendication 21, contenant du D-mannitol en tant qu'excipient.

23. Procédé pour fabriquer une formulation solide selon l'une quelconque des revendications 1 à 22, dans lequel la formulation solide est un ou plusieurs choisis dans le groupe constitué par un comprimé, un granule, une poudre et une capsule.

24. Formulation solide produite par le procédé de fabrication selon l'une quelconque des revendications 1 à 23.

25. Formulation solide selon la revendication 24, contenant un composé instable vis-à-vis de l'eau, son sel pharmaceutiquement acceptable ou son solvate, en tant que principe actif, et un excipient, dans laquelle la teneur en le principe actif est de 1 % ou moins et l'écart type de la teneur en le principe actif est de 3 % ou moins dans la formulation, dans laquelle le principe actif est un composé représenté par la formule (IA) : 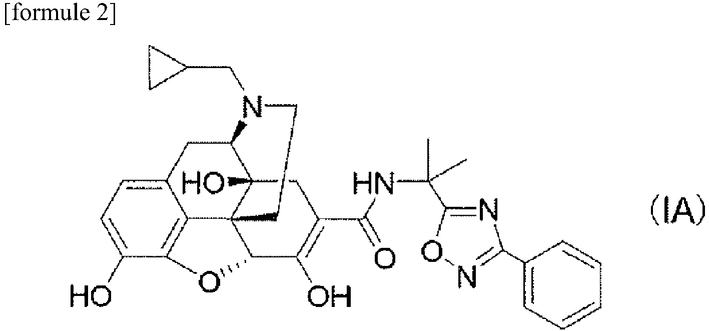 son sel pharmaceutiquement acceptable, ou un solvate de celui-ci.

26. Formulation solide selon la revendication 25, dans laquelle la teneur en l'excipient est de 70 à 99,99 % en poids dans la formulation.
